# EUROPÄISCHE PATENTSCHRIFT

(11) **EP 0 603 751 B1**
(45) Veröffentlichungstag und Bekanntmachung des Hinweises auf die Patenterteilung: **24.05.2000**
(21) Anmeldenummer: 93120352.5
(22) Anmeldetag: 17.12.1993
(51) Int. Cl.: G01N 33/18, G01N 31/00, G01N 27/06

(54) **Verfahren zum Nachweisen von Verbindungen, die in einer mindestens einen Feststoff enthaltenden flüssigen Phase vorliegen.**
Method for detecting compounds which are present in a liquid phase comprising a solid
Méthode pour la détection de composés présents dans une phase liquide comportant un solide

(30) Priorität: 21.12.1992 DE 4243121
(43) Veröffentlichungstag der Anmeldung: 29.06.1994
(73) Patentinhaber: Degussa-Hüls Aktiengesellschaft, 60287 Frankfurt am Main (DE)
(72) Erfinder: Duve, Hans, D-48249 Dülmen (DE); Adam, Horst, D-45772 Marl (DE); Stucke, Astrid, D-45721 Haltern (DE)
(74) Vertreter: Olbricht, Gerhard, Dr.

(56) Entgegenhaltungen:
- EP-A- 0 422 572
- WO-A-91/13362
- DE-A- 3 223 167
- DE-A- 3 842 068

## Beschreibung

Die Erfindung betrifft ein Verfahren und eine Vorrichtung zum Nachweisen von Verbindungen, die in einer mindestens einen Feststoff enthaltenden flüssigen Phase vorliegen, und bei deren Reaktion mit Chemikalien flüchtige Verbindungen entstehen.

Sie verfolgt den Zweck, derartige Verbindungen auf einfache Weise nachzuweisen, auch wenn es sich bei der mindestens einen Feststoff enthaltenden flüssigen Phase z. B. um eine Suspension handelt.

Bei den bekannten Nachweisverfahren werden unterschiedliche Methoden angewendet, um Verbindungen in einer flüssigen Phase nachzuweisen. Bei den maßanalytischen Verfahren wird der Verbrauch an Säure- oder Laugelösung mit bekannten Konzentrationen für eine definierte Probenmenge verwendet. Liegt die nachzuweisende Verbindung allein in einer wäßrigen Phase vor, oder führen weitere in der wäßrigen Phase enthaltene Verbindungen nicht zu Querempfindlichkeiten, werden physikalisch-chemische Parameter wie die spezifische Leitfähigkeit oder der pH-Wert verwendet.

Für die Untersuchung von Carbonat (CO₃⁻⁻) und/oder Sulfit (SO₃⁻⁻) enthaltenden Suspensionen werden spezielle Untersuchungsverfahren beschrieben. In DE-34 29 952 wird ein Verfahren zur kontinuierlichen Bestimmung der CaCO₃- und CaSO₃-Konzentration einer Aufschlämmung angegeben, bei dem die Aufschlämmung in einem Reaktor mit Säure versetzt wird, Luft oder Stickstoff eingeblasen wird und die im abgezogenen Gas enthaltene CO₂- bzw. SO₂-Konzentration mit Hilfe von Gasanalysatoren bestimmt wird. Bei diesem Verfahren ist es erforderlich, die Reaktionen mit Säure bei Temperaturen von 70 °C durchzuführen, die Gasdurchsatzmenge genau zu messen und zur CO₂-/SO₂ -Bestimmung im Gasstrom aufwendige und störanfällige Analysatoren einzusetzen. Nach DE-34 29 951 wird dieses Verfahren bei einem pH-Wert unterhalb 4 durchgeführt.

Aus DE-38 09 379 ist ein Verfahren zur Bestimmung des Carbonatgehaltes einer Kalksteinsuspension bekannt, bei welchem zu einer Suspensionsprobe in einem geschlossenen Gefäß Säure zugegeben und das Volumen des entstehenden CO₂-Gases gemessen wird. Bei diesem Verfahren kann der CaSO₃-Gehalt einer Suspension nicht bestimmt werden. Wird das Verfahren zur CaCO₃ -Bestimmung verwendet, ist man bei der Volumenmessung auf das Konstanthalten von Temperatur und Druck angewiesen.

In DE-34 05 580 wird ein Verfahren zur kontinuierlichen Bestimmung der Konzentration an Carbonat oder Sulfit in einer Flüssigkeit angegeben, wobei man eine Carbonat- und/oder Sulfit-haltige Flüssigkeit kontinuierlich einem Gefäß zuführt, die Flüssigkeit durch Schwefelsäure auf einem pH-Wert nicht über 3 hält, das gebildete CO₂ und/oder SO₂ mittels Luft als Trägergas in ein Auffanggefäß leitet, die Konzentration an CO₂ und/oder SO₂ im Trägergas bestimmt und daraus die Konzentration an Carbonat und/oder Sulfit in der Flüssigkeit berechnet.

Aus EP-0 424 799 ist ein Verfahren zur Bestimmung des Carbonatgehalts von Waschsuspensionen von mit Kalkstein betriebenen Rauchgasentschwefelungsanlagen bekannt, bei dem die warmen Proben zunächst mit einem Überschuß an Eisen-III-chlorid versetzt werden, nach der Oxidation des Sulfits zu Sulfat Säure im Überschuß zugegeben wird und die dem Carbonatgehalt entsprechende CO₂-Menge in der Gasphase gemessen wird.

Bei den letzten beiden Verfahren sind die für die Gasanalyse erforderlichen Randbedingungen sorgfältig einzuhalten.

Aus DE-32 23 167 ist ein Verfahren zum kontinuierlichen Untersuchen von Wasser, das zersetzungsfähige Kohlenstoff-Verbindungen enthält, bekannt, bei dem unter anderem die anorganischen Kohlenstoff-Verbindungen (Carbonate) mittels Säure zersetzt werden, das dabei erzeugte CO₂ mittels eines Trägergases in eine mit Wasser gefüllte Leitfähigkeitsmeßzelle geleitet und dort die Leitfähigkeit kontinuierlich gemessen wird. Wegen der dortigen Randbedingungen ist dieses Verfahren bei Suspensionen oder Aufschlämmungen praktisch nicht verwendbar.

Aus DE 38 42 068 ist ein Verfahren zur Bestimmung des Ammoniumgehaltes in wäßrigen Systemen, insbesondere zur Gewässerüberwachung und zur Steuerung von Nitrifikations-Denitrifikationsprozessen in Reinigungsanlagen, bekannt, bei dem einem mengenmäßig variablen, in Abhängigkeit vom NH₃-Gehalt genau bestimmten Teil der zu untersuchenden Flüssigkeit in einem Strippreaktor bis zu einem festgelegtem pH-Wert diskontinuierlich Lauge oder eine Pufferflüssigkeit zugegeben wird und bei dem das unter Rühren entstehende NH₃-Gas mit Hilfe eines von CO₂ gereinigten Strippgases, welches keinen Einfluß auf den pH-Wert nimmt, ausgestrippt und in einen Wasser enthaltenden Meßreaktor überführt wird. Die Bestimmung des in der zu untersuchenden Flüssigkeit enthaltenen Ammoniumgehaltes geschieht im Meßreaktor durch Bestimmung der elektrischen Leitfähigkeit des Wassers und anschließender Berechnung der Ammoniumkonzentration aus der gemessenen elektrischen Leitfähigkeit. Nach jeder Probenahme werden in einem Reinigungszyklus Ventile in den Auslaufleitungen des Strippreaktors geöffnet. Besonders wegen der Ausführung der Probenentnahme ist dieses Verfahren zur Untersuchung von Suspensionen oder Aufschlämmungen ungeeignet, da es bei der Untersuchung von feststoffhaltigen Flüssigkeiten zu Meßungenauigkeiten kommen kann, wenn die in der Flüssigkeit enthaltenen Feststoffe nicht vollständig in den Reaktionsbehälter überführt werden. Erhebliche Probleme bringt außerdem die diskontinuierliche Fahrweise des Strippreaktors mit sich, da es in den Leitungssystemen durch die über Ventile gesteuerte diskontinuierliche Fahrweise bei der Anwendung des Verfahrens auf feststoffhaltige flüssige Phasen zu Verstopfungen oder Undichtigkeiten kommen kann.

Damit stellt sich die Aufgabe, ein Verfahren und eine Vorrichtung zu finden, mit denen man Verbindungen, die in einer flüssigen Phase vorliegen, mit hoher Empfindlichkeit und geringem Aufwand nachweisen kann, auch dann, wenn es sich bei der flüssigen Phase z. B. um eine Suspension oder Aufschlämmung handelt, oder die flüssige Phase weitere Stoffe, z. B. Schmutz oder Ruß, enthält, die die Anwendung bekannter Nachweisverfahren erschweren oder verhindern. Das Verfahren wird auf mindestens einen Feststoff enthaltende flüssige Phasen angewendet.

Diese Aufgabe wird erfindungsgemäß gelöst durch ein Verfahren zum Nachweis mindestens einer Verbindung, die in einer mindestens einen Feststoff enthaltenden flüssigen Phase vorliegt, angewendet auf flüssige Phasen, wobei
- ein Teil der mindestens einen Feststoff enthaltenden flüssigen Phase in einen Reaktionsbehälter gegeben wird,
- in den Reaktionsbehälter Chemikalien eingegeben werden,
- zumindest eine nachzuweisende Verbindung im Reaktionsbehälter in eine flüchtige Verbindung umgewandelt wird,
- mindestens eine der im Reaktionsbehälter vorhandenen flüchtigen Verbindungen mittels eines Trägergases ausgetrieben wird,
welches dadurch gekennzeichnet ist,
daß eine definierte Probenmenge der zu untersuchenden flüssigen Phase mit der im Kreis gepumpten Flüssigkeit des Reaktionsbehälters aus einer Probeentnahmevorrichtung in den Reaktionsbehälter gespült wird und daß das die flüchtigen Verbindungen enthaltende Trägergas kontinuierlich in ein Absorptionsgefäß übergeleitet wird, zumindest eine der im Trägergas enthaltenen flüchtigen Verbindungen in einem Absorptionsgefäß in Wasser niedriger Leitfähigkeit absorbiert wird und die im Wasser enthaltende flüchtige Verbindung, die ein Maß für die in der mindestens einen Feststoff enthaltenden flüssigen Phase vorliegende nachzuweisende Verbindung ist, durch Messen der Leitfähigkeit oder mittels einer ionensensitiven Elektrode kontinuierlich nachgewiesen wird.

Die Reihenfolge des Eingebens der Teilmenge der mindestens einen Feststoff enthaltenden flüssigen Phase und der Chemikalien in den Reaktionsbehälter kann unterschiedlich sein.

Nach dem Umwandeln der nachzuweisenden Verbindung, die in vielen Fällen qualitativ bereits bekannt ist, liegt mindestens eine flüchtige Verbindung vor. Diese kann aus der nachzuweisenden Verbindung entstanden sein oder aus der im Überschuß eingegebenen Chemikalie. In beiden Fällen ist die Masse der flüchtigen Verbindung ein Maß für die Masse der nachzuweisenden Verbindung in der vorgelegten mindestens einen Feststoff enthaltenden flüssigen Phase.

In einem weiteren Fall entsteht die flüchtige Verbindung zwar ebenfalls aus der eingegebenen Chemikalie. Die nachzuweisende Verbindung verringert jedoch durch Reaktion die Konzentration der aus der eingegebenen Chemikalie entstandenen flüchtigen Verbindung. In diesem Fall ist die Masse der flüchtigen Verbindung reziprok-proportional zur Masse der nachzuweisenden Verbindungen in der vorgelegten mindestens einen Feststoff enthaltenden flüssigen Phase, wenn die Chemikalie im Überschuß und in konstanter Menge eingegeben wird.

Die in der mindestens einen Feststoff enthaltenden flüssigen Phase vorliegende nachzuweisende Verbindung kann eine anorganische oder organische Verbindung sein; sie kann aus nur einem Stoff bestehen, oder es können mehrere Stoffe nebeneinander vorliegen.

Zum Umwandeln der nachzuweisenden Verbindung werden Säure, Lauge, Salz, Oxidationsmittel und/oder Reduktionsmittel in den Reaktionsbehälter gegeben. Die flüchtige Verbindung ist z. B. CO₂, SO₂, NH₃, NO, NO₂, HCN, PH₃, Cl₂, F₂, H₂S; diese wird mittels des vorzugsweise durch die flüssige Phase im Reaktionsbehälter hindurch strömenden Trägergases aus der flüssigen Phase praktisch vollständig ausgetrieben und geht in das Trägergas über. Die flüchtige Verbindung kann aus nur einem oder aus mehreren Gasen bestehen.

Als Trägergas ist jedes Gas geeignet, das sich gegenüber den im Reaktionsbehälter anwesenden Stoffen und gegenüber der flüchtigen Verbindung inert verhält, und das das Analysieren der im Trägergas enthaltenen flüchtigen Verbindung nicht stört, bevorzugt Luft, Stickstoff, Wasserstoff oder ein Edelgas. Das Trägergas muß vor dem Eintreten in die flüssige Phase im Reaktionsbehälter frei sein von der im Reaktionsbehälter entstehenden flüchtigen Verbindung, oder es muß diese in zumindest konstanter Konzentration enthalten.

Die Gasphase, bestehend aus dem Trägergas und der darin enthaltenen flüchtigen Verbindung wird in ein Absorptionsgefäß geleitet, das . Wasser mit niedriger Leitfähigkeit enthält.

Die im Absorptionsmittel absorbierte flüchtige Verbindung wird analysiert. Die Änderung der Leitfähigkeit oder des pH-Wertes des Wassers wird gemessen.

In allen Fällen wird aus der Masse der flüchtigen Verbindung die Masse der in der mindestens einen Feststoff enthaltenden flüssigen Phase vorliegenden Verbindung berechnet.

Die mindestens einen Feststoff enthaltenden flüssige Phase, die die nachzuweisende Verbindung enthält, sowie die für das Umwandeln dieser Verbindung eingesetzten Chemikalien können kontinuierlich in den Reaktionsbehälter eingegeben werden. Die flüchtige Verbindung wird kontinuierlich durch das Trägergas ausgetrieben und nach dem Absorbieren kontinuierlich analysiert. Für die Untersuchung von Einzelproben wird die Teilmenge der mindestens einen Feststoff enthaltenden flüssigen Phasen diskontinuierlich in den Reaktionsbehälter eingegeben. Bei einer im Reaktionsbehälter schnell ablaufenden Reaktion und bei einem schnellen Analyseverfahren für die absorbierte flüchtige Verbindung kann das diskontinuierliche Verfahren quasi-kontinuierlich werden.

Je nach Art der nachzuweisenden Verbindung werden unterschiedliche Chemikalien mit speziellen Eigenschaften der mindestens einen Feststoff enthaltenden flüssigen Phase zugegeben, um bei der Reaktion mit der nachzuweisenden Verbindung mindestens eine flüchtige Verbindung zu erzeugen, oder um die Masse der entstehenden flüchtigen Verbindung zu verringern.

Enthält die mindestens einen Feststoff enthaltende flüssige Phase z. B. Carbonate (CO₃⁻⁻), Sulfite (SO₂⁻⁻) oder Cyanide (CN⁻), entstehen durch Eingeben einer Säure wie Schwefelsäure, Phosphorsäure oder Salzsäure die flüchtigen Verbindungen Kohlendioxid (CO₂), Schwefeldioxid (SO₂) beziehungsweise Blausäure (HCN).

Enthält die mindestens einen Feststoff enthaltende flüssige Phase z. B. Säure, gegebenenfalls verschiedene Säuren nebeneinander, entsteht durch Eingeben von Carbonat (CO₃⁻⁻) die flüchtige Verbindung Kohlendioxid (CO₂). Das CO₂ stammt in diesem Fall aus dem eingegebenen Carbonat; seine Masse ist äquivalent zu der in der flüssigen Phase nachzuweisenden Masse an Wasserstoff-Ionen (H⁺).

Enthält die mindestens einen Feststoff enthaltende flüssige Phase z. B. Ammonium-Salze, entsteht durch Eingeben von Natronlauge die flüchtige Verbindung Ammoniak (NH₃).

Enthält die mindestens einen Feststoff enthaltende flüssige Phase z. B. Sulfid, wie suspendiertes Eisensulfid (FeS), entsteht durch Eingeben von Salzsäure die flüchtige Verbindung Schwefelwasserstoff (H₂S).

Enthält die mindestens einen Feststoff enthaltende flüssige Phase z. B. Carbonat (CO₃⁻⁻) und Sulfit (SO₃⁻⁻) nebeneinander, und soll nur die Menge an Carbonat nachgewiesen werden, wird ein Oxidationsmittel, z. B. Wasserstoffperoxid (H₂O₂), zwecks Oxidation des Sulfits zu Sulfat (SO₄⁻⁻) eingegeben, und gleichzeitig oder anschließend wird Phosphorsäure eingeben, mit der aus dem Carbonat die flüchtige Verbindung CO₂ erzeugt wird. Hierbei entsteht kein SO₂ aus dem Sulfit.

Enthält die mindestens einen Feststoff enthaltende flüssige Phase z. B. Ozon (O₃) oder Wasserstoffperoxid (H₂O₂), so wird ein Teil des mit konstanter Menge eingegebenen Sulfits (SO₃⁻⁻) als Chemikalie unter sauren Bedingungen zu Sulfat (SO₄⁻⁻) oxidiert. Je größer die Konzentration an Oxidationsmittel in der mindestens einen Feststoff enthaltenden flüssigen Phase ist, um so geringer ist die verbleibende Konzentration der flüchtigen Verbindung SO₂.

Um die Löslichkeit der entstehenden flüchtigen Verbindung in der Flüssigkeit des Reaktionsbehälters zu verringern, kann es vorteilhaft sein, die Temperatur im Reaktionsbehälter zu erhöhen, gegebenenfalls bis zur Siedetemperatur der flüssigen Phase.

Zum optimalen Verteilen der eingegebenen Chemikalien, des Trägergases sowie der gegebenenfalls in der Flüssigkeit im Reaktionsbehälter vorhandenen ungelösten Feststoffe kann es vorteilhaft sein, diese Flüssigkeit ständig zu rühren, z. B. mit einem Magnetrührer. Zur Erhöhung der Meßgenauigkeit ist die Drehzahl des Rührers konstant zu halten.

Die im Reaktionsbehälter enthaltene Flüssigkeit wird nicht nur gerührt, sondern auch im Kreislauf umgepumpt. Die zu untersuchende mindestens einen Feststoff enthaltende flüssige Phase, z. B. eine Suspension oder Aufschlämmung, wird unter Verwenden der im Kreislauf geführten Flüssigkeit im Reaktionsbehälter in den Behälter eingespült. Enthält die Flüssigkeit im Reaktionsbehälter unter den gegebenen Bedingungen Feststoffe, die an der Reaktion nicht teilnehmen, kann es vorteilhaft sein, die Flüssigkeit über ein Filter ständig oder teilweise im Kreislauf umzupumpen, um derartige Feststoffe aus der Flüssigkeit zu entfernen.

In manchen Fällen genügt es, die für das Umwandeln der nachzuweisenden Verbindung eingesetzten Chemikalien in flüssiger oder fester Form an einer beliebigen Stelle in den Reaktionsbehälter einzugeben. In speziellen Fällen ist es zweckmäßig, die Chemikalie in flüssiger Form in die Zuleitung für das Trägergas einzugeben und sie mit diesem in den Reaktionsbehälter einzuspülen und zu verteilen.

Das Trägergas kann aus einem Vorratsbehälter in die im Reaktionsbehälter vorhandene Flüssigkeit, bevorzugt mit Hilfe einer Verteilungsvorrichtung, z. B. einer Glasfritte, eingegeben werden und nach dem Verlassen des Reaktionsbehälters und des angeschlossenen Absorptionsgefäßes verworfen werden. Es ist jedoch zweckmäßig, das Trägergas im Kreislauf umzupumpen, d. h. nach dem Verlassen des Absorptionsgefäßes in den Reaktionsbehälter zurückzuführen. Dabei kann es zweckmäßig sein, Reste der flüchtigen Verbindung aus dem Trägergas abzutrennen, z. B. durch Kondensieren oder Ausfrieren in einer Kühlfalle, durch Auswaschen mit einer Waschflüssigkeit oder durch Absorbieren an einem Festbettabsorber.

Entweder kann das Wasser mit geringer Leitfähigkeit in einem konstanten Strom durch das Absorptionsgefäß geleitet werden, oder das Wasser kann im Kreislauf umgepumpt werden. Im letzten Fall kann es zweckmäßig sein, das Wasser durch einen Ionenaustauscher zu leiten, der die im Wasser enthaltenen Ionen entfernt. Das Kreislaufwasser kann mit einer Geschwindigkeit von Null bis 100 Meter pro Stunde umgepumpt werden. d. h. die Umwälzpumpe kann auch abgeschaltet werden.

Falls eine einzige mindestens einen Feststoff enthaltende flüssige Phase über lange Zeit untersucht werden soll und während dieser Zeit die Art der nachzuweisenden Verbindung unverändert bleibt und sich nur deren Konzentration ändert, kann die mindestens einen Feststoff enthaltende flüssige Phase z. B. mittels einer Dosierpumpe mit konstantem Durchsatz durch den Reaktionsbehälter gepumpt werden. Gleichzeitig wird ein hinreichend großer konstanter Strom von Chemikalien in den Reaktionsbehälter eingegeben. Der bevorzugt im Kreislauf umgepumpte Trägergasstrom durch den Reaktionsbehälter wird hinreichend groß gewählt, um die erzeugte flüchtige Verbindung praktisch vollständig aus der im Reaktionsbehälter enthaltenen Flüssigkeit auszutreiben.

Enthält die mindestens einen Feststoff enthaltende flüssige Phase nur eine sehr geringe Konzentration der nachzuweisenden Verbindung, kann es zweckmäßig sein, auf das Abtrennen von Resten der flüchtigen Verbindung aus dem das Absorptionsgefäß verlassenden Trägergas für die Dauer einer Analyse zu verzichten.

Die Vorrichtung zur Durchführung des Verfahrens enthält mindestens einen Reaktionsbehälter, der mit je einem Einlaß für die mindestens einen Feststoff enthaltende flüssige Phase, die Chemikalien und das Trägergas sowie mit je einem Auslaß für die im Reaktionsbehälter vorhandene Flüssigkeit und für das Trägergas versehen ist, und der über eine Trägergasleitung mit dem Absorptionsgefäß verbunden ist. Die Vorrichtung kann eine weitere Trägergasleitung enthalten, die vom Absorptionsgefäß zum Reaktionsbehälter zurückführt, und die gegebenenfalls eine Vorrichtung zum Abtrennen von Resten der flüchtigen Verbindung aus dem Trägergas enthält. Die Abtrennvorrichtung kann z. B. eine Kühlfalle, eine Waschflasche oder ein Festbettabsorber sein.

Weiter kann ein Wasserkreislauf vorgesehen werden, der einen Ionenaustauscher enthält.

Dem Reaktionsbehälter ist eine Probenahmevorrichtung vorgeschaltet, die z. B. einen umschaltbaren Durchgangshahn enthält.

Ist aus einer Leitung, die z. B. eine Kalkstein-Suspension aus einer Rauchgasentschwefelungsanlage enthält, eine repräsentative Probe zu entnehmen, kann es zweckmäßig sein, die aus dieser Leitung abgezweigte Leitung einschließlich des Durchgangshahnes zwischen zwei Probenahmen - bevorzugt im Gegenstrom - zu spülen, um Ablagerungen oder Verstopfungen zu verhindern.

Häufig ist es für die Überwachung eines (groß-)technischen Prozesses sowie für dessen Regelung oder Steuerung hinreichend, wenn der als Führungsgröße dienende Analysenwert diskontinuierlich - gegebenenfalls in größeren Zeitabständen - aktualisiert wird Dann können einem Reaktionsbehälter mehrere Probenahmenvorrichtungen vorgeschaltet werden, aus denen abwechselnd Einzelproben in den Reaktionsbehälter eingegeben und untersucht werden. Die mehrfach vorhandenen Probenahmevorrichtungen können an unterschiedliche Stellen des (groß-)technischen Prozesses angeschlossen sein. Weiter kann es zweckmäßig sein, an die Leitung für die zu untersuchende mindestens einen Feststoff enthaltende flüssige Phase zwei voneinander unabhängig arbeitende Probenahmevorrichtungen anzuschließen

Das erfindungsgemäße Nachweisverfahren besteht aus der Probenahmestufe, der Reaktionsstufe, der Absorptionsstufe und der Analysenstufe; nachgeschaltet ist eine Regel- und/oder Steuerstufe. Die Analysenstufe enthält z. B. die Meßwerterfassung, Registrierung, Integration, Umrechnung über eine oder mehrere Kalibrierkurven, Speicherung und Dokumentation, Zuordnung der Meßergebnisse zu den vorgelegten Proben, Ausgeben der Führungsgröße. Die Probenahmestufe kann mehrere Probenahmevorrichtungen enthalten. Die Reaktionsstufe, die Absorptionsstufe und die Analysenstufe können - gemeinsam oder einzeln - mehrfach vorhanden sein. Die Proben können - gegebenenfalls gleichzeitig nebeneinander - kontinuierlich oder diskontinuierlich entnommen werden. Dieses gesamte Verfahren läßt sich über eine frei programmierbare Multifünktionseinheit (z. B. SIPART DR 24; Siemens) praktisch vollständig automatisieren.

Das erfindungsgemäße Verfahren hat folgende Vorteile:
- Es ist anwendbar auf Suspensionen, Emulsionen, Aufschlämmungen und ungereinigte mindestens einen Feststoff enthaltende flüssige Phasen wie Abwasser.
- Aufwendige Aufbereitungsschritte der mindestens einen Feststoff enthaltenden flüssigen Phase sind nicht erforderlich; ohne derartige Schritte sind die bekannten Nachweismethoden entweder nicht anwendbar, oder sie ergeben unzuverlässige Ergebnisse.
- Je nach angewendetem Analysenverfahren dauert es nur etliche Sekunden bis wenige Minuten nach dem Eingeben der mindestens einen Feststoff enthaltenden flüssigen Phase in den Reaktionsbehälter, bis das Analysenergebnis vorliegt.
- Die mindestens einen Feststoff enthaltende flüssige Phase kann wahlweise kontinuierlich oder diskontinuierlich untersucht werden.
- Es ist als kontinuierliches oder quasi-kontinuierliches Verfahren für die Prozeßregelung geeignet.
- Die in den Reaktionsbehälter eingegebenen Chemikalien müssen im allgemeinen nicht exakt dosiert werden, da sie stets im Überschuß im Reaktionsbehälter vorliegen.
- Über die Größe der Stoffströme (Strom der Teilmenge der flüssigen Phase, des Trägergases und des Wassers über den Ionenaustauscher im Wasserkreislauf) sowie über das wahlweise Abtrennen von Resten der flüchtigen Verbindung aus dem Trägergasstrom hinter dem Absorptionsgefäß kann die in der mindestens einen Feststoff enthaltenden flüssigen Phase enthaltene Verbindung in einem sehr großen Konzentrationsbereich nachgewiesen werden, der von etlichen Prozent bis in den ppb-Bereich reicht.
- Durch die interne Kreislaufführung des Trägergases und des Wassers im Absorptionsgefäß über einen Ionenaustauscher ist das Verfahren sehr einfach und unanfällig gegenüber Störungen.
- Es ist kalibrierfähig, wenn die nachzuweisende Verbindung qualitativ bekannt ist. Die Kalibrierung bleibt bei monatelangem Dauerbetrieb unverändert.
- Der Ablauf des gesamten Nachweisverfahrens einschließlich der Steuerung des zu überwachenden Prozesses läßt sich durch eine frei programmierbare Steuerung praktisch vollständig automatisieren.

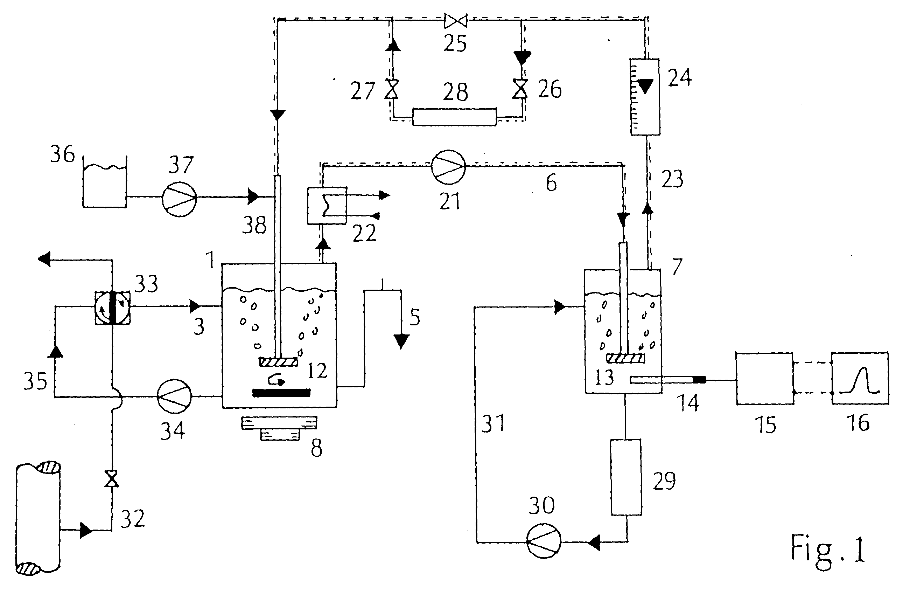

Das erfindungsgemäße Verfahren kann beispielsweise mittels der in der Figur 1 dargestellten Vorrichtung durchgeführt werden.

Figur 1 zeigt den Reaktionsbehälter (1) mit Zuleitung (38) für das Trägergas, Zuleitung (3) zum Eingeben der mindestens einen Feststoff enthaltenden flüssigen Phase, die die nachzuweisende Verbindung enthält, und eine Zuleitung zum Eingeben der Chemikalien. Aus dem Überlauf (5) läuft die Flüssigkeit aus dem Reaktionsbehälter ab, sobald sie die konstante Sollhöhe erreicht hat. Über die aus dem Gasraum des Reaktionsbehälters abgehende Leitung (6) wird das Trägergas dem Absorptionsgefäß (7) zugeführt. Die Flüssigkeit im Reaktionsbehälter wird mit dem Magnetrührer (8) gerührt. An den Enden der Gaseinleitungen im Reaktionsbehälter und im Absorptionsgefäß sind jeweils eine Gasfritte (12) und (13) angebracht. In das Wasser im Absorptionsgefäß taucht eine Leitfähigkeitselektrode (14) ein, die an die Auswerteeinheit (15) und den Schreiber (16) angeschlossen ist.

Die Trägergasleitung (6) enthalt die Kreisgaspumpe (21) und den Gaskühler (22). Die Rücklaufleitung (23) für das Trägergas enthält einen Druckflußmesser (24), das Ventil (25) sowie eine Bypass-Leitung mit den Ventilen (26) und (27) und der Abtrennvorrichtung (28). Das Wasser im Absorptionsgefäß (7) wird über den Ionenaustauscher (29) mittels der Pumpe (30) und der Leitung (31) in das Absorptionsgefäß (7) zurückgeführt.

Aus der Leitung, die die zu untersuchende mindestens einen Feststoff enthaltende flüssige Phase enthält, zweigt die Leitung (32) ab, die zu dem Durchgangshahn (33) führt. Die Flüssigkeit im Reaktionsbehälter wird mittels der Spülpumpe (34) über die Spülleitung (35) zum Durchgangshahn (33) geführt und über die Leitung (3) in den Reaktionsbehälter zurückgeleitet. Der Behälter (36) enthält die Chemikalien, die über die Dosierpumpe (37) in die Leitung (38) für das zurückgeführte Trägergas eingegeben werden.

Der umschaltbare Durchgangshahn (33) für die Probenahme hat vier Anschlüsse. An zwei einander gegenüberliegenden Anschlüssen ist die Leitung (32) angeschlossen, an die anderen beiden einander gegenüberliegenden Anschlüsse ist die Spülleitung (35) beziehungsweise die Leitung (3) angeschlossen. Der Durchgangshahn enthält ein drehbares Küken mit einem bestimmten freien Volumen. Durch Drehen des Kükens kann dieses Volumen von der Leitung (32) in die Leitung (35) gelegt werden.

Das erfindungsgemäße Verfahren wird durch folgende Beispiele weiter erläutert.

### Beispiel 1: Bestimmung des Carbonatgehalts einer Suspension

Die wäßrige Suspension in einer unter Druck stehenden Leitung hat folgende Zusammensetzung:
15 g/l Calciumcarbonat (CaCO₃)
23 g/l Calciumsulfat (CaSO₄·2 H₂O)
60 g/l Calciumsulfit (CaSO₃·½ H₂O)
5 g/l Chlorid (Cl⁻)

Ein Seitenstrom dieser Suspension wird über die Leitung (32) durch den Durchgangshahn (33) geführt, der ein freies Volumen von 0,8 ml hat. Der Trägergasstrom durch den Reaktionsbehälter (1), die Kreisgasleitungen (6) und (23), durch das Absorptionsgefäß (7) und über die Abtrennvorrichtung (28) bei geschlossenem Ventil (25) und geöffneten Ventilen (26) und (27) beträgt 100 Liter pro Stunde. Die Abtrennvorrichtung ist in diesem Fall ein CO₂-Absorber.

Im Reaktionsbehälter (1) werden 0,6 Liter entionisiertes Wasser und im Absorptionsgefäß (7) 0,3 Liter Wasser mit niedriger Leitfähigkeit vorgelegt. Mit der Pumpe (37) werden 0,05 Liter pro Stunde einer wäßrigen Lösung, die 1 Massen-% Phosphorsäure (H₃PO₄) und 0,1 Massen-% Wasserstoffperoxid (H₂O₂) enthält, aus dem Chemikalienbehälter (36) über die Trägergaseinleitung (38) in den Reaktionsbehälter (1) gefördert.

Vor Beginn der Analyse wird das Kreisgas (Luft) etwa 5 Minuten lang durch den CO₂-Absorber (28) gepumpt und von CO₂ befreit.

Das Wasser im Absorptionsgefäß (7) wird mittels der Pumpe (30) über den Ionenaustauscher (29) gefahren, hier von ionogenen Inhaltsstoffen befreit und mit 2 Liter pro Stunde über die Wasser-Kreislaufleitung (31) in das Absorptionsgefäß (7) zurückgeführt. Vor Beginn der Analyse beträgt die Leitfähigkeit des Wassers im Absorptionsgefäß (7) 0,1 µS/cm.

Durch eine 90 Grad-Drehung des Durchgangshahnes (33) wird das mit der zu untersuchenden Suspension gefüllte freie Volumen (0,8 ml) aus der Abzweigleitung (32) herausgeschaltet und wird somit Teil der Spülleitung (35). Die Spülpumpe (34) saugt unten am Reaktionsbehälter (1) befindliche Flüssigkeit an und transportiert über die Spülleitung (35) die im Durchgangshahn (33) enthaltene Suspensionsprobe (0,8 ml) in den Reaktionsbehälter (1). Die Flüssigkeit im Reaktionsbehälter hat einen pH-Wert von kleiner als 2 und enthält einen Überschuß an Wasserstoffperoxid und Phosphorsäure.

Das in der Suspensionsprobe enthaltene Sulfit (SO₃⁻⁻) wird durch Wasserstoffperoxid zu Sulfat (SO₄⁻⁻) oxidiert, bevor sich gasförmiges Schwefeldioxid (SO₂) bilden kann. Das Wasserstoffperoxid verhindert das Umwandeln von Sulfit-Ionen in flüchtiges Schwefeldioxid. Das in der Suspensionsprobe enthaltene Carbonat (CO₃⁻⁻) wird unter den im Reaktionsbehälter (1) vorliegenden Bedingungen in gasförmige Kohlendioxid (CO₂) umgewandelt.

Das flüchtige CO₂-Gas wird vom Trägergas aus der Flüssigkeit im Reaktionsbehälter ausgetrieben und aus dem Reaktionsbehälter (1) in das Absorptionsgefäß (7) transportiert. Hier löst sich das CO₂ teilweise im Wasser und erhöht dessen Leitfähigkeit, die durch die Leitfähigkeitselektrode (14) mit Auswerteeinheit (15) detektiert und ausgewertet wird.

Das Trägergas wird über den CO₂-Absorber (28) zum Reaktionsbehälter (1) zurückgeführt. Im CO₂-Absorber werden Reste von CO₂ aus dem Trägergas abgetrennt. Das im Wasser der Meßzelle gelöste CO₂ wird durch den Ionenaustauscher (29) aus dem im Kreislauf gefahrenen Wasser abgetrennt.

Etwa 10 Sekunden nach dem Eingeben der Suspensionsprobe in den Reaktionsbehälter steigt die Leitfähigkeit im Absorptionsgefäß an. Der Grundwert von 0,1 µS/cm erhöht sich innerhalb von 90 Sekunden auf 1,5 µS/cm und kehrt innerhalb von weiteren 200 Sekunden auf den Grundwert zurück.

Nach dem Erreichen des Maximums der Leitfähigkeit (etwa 100 Sekunden nach der Probenahme) wird der Durchgangshahn wieder umgeschaltet; nun strömt wieder die Suspension durch die Abzweigleitung und den Durchgangshahn.

Sowohl das Maximum der peakförmigen Leitfähigkeitserhöhung als auch das Flächenintegral werden von der Auswerteeinheit berechnet und gespeichert. Das Flächenintegral und die Peakhöhe sind ein Maß für den Carbonat-Gehalt der zu untersuchenden Suspension.

Der Nachweis von Carbonat wird durch Salzionen bei diesem Verfahren nicht gestört.

### Beispiel 2: Bestimmung des Carbonatgehalts einer Suspension (Endwertverfahren)

Vor Beginn der Untersuchung werden das Trägergas (Luft) und das Wasser im Absorptionsgefäß etwa 5 Minuten durch den CO₂-Absorber (28) beziehungsweise den Ionenaustauscher (29) gefahren und darin von Resten von CO₂ befreit. Anschließend wird in Abänderung von Beispiel 1 das Ventil (25) in der Trägergasrückleitung geöffnet, und die Ventile (26) und (27) werden geschlossen. Nachdem die Pumpe (30) das Wasser im Absorptionsgefäß (7) etwa 5 Minuten lang durch den Ionenaustauscher (29) gepumpt hat, wird die Pumpe (30) abgeschaltet.

Nach dem Eingeben - analog zu Beispiel 1 - einer 0,8 ml Suspensionsprobe steigt die Leitfähigkeit innerhalb von etwa 3 Minuten von 0,1 µS/cm auf 4,5 µS/cm an und bleibt konstant.

Der Endwert der Leitfähigkeit ist ein Maß für den Carbonatgehalt der untersuchten Suspension.

Nach dem Einschalten der Pumpe (30), Öffnen der Ventile (26) und (27) und Schließen des Ventils (25) sinkt die Leitfähigkeit des Wassers im Absorptionsgefäß wieder auf 0,1 µS/cm.

### Beispiel 3: Bestimmung des Sulfitgehalts einer Suspension

Die Suspension hat folgende Zusammensetzung:
0,5 g/l Calciumsulfit (CaSO₃ · ½ H₂O)
14,0 g/l Calciumsulfat (CaSO₄ · 2 H₂O)
weniger als 0,01 g/l Calciumcarbonat (CaCO₃)
3,0 g/l Chlorid (Cl⁻)

Man geht analog zu Beispiel 1 vor, jedoch enthält der Chemikalienbehälter (36) nun eine wäßrige Lösung von Phosphorsäure ohne Wasserstoffperoxid. Die Flüssigkeit im Reaktionsbehälter ist ebenfalls frei von Oxidationsmitteln. Durch das Absorptionsgefäß (7) werden - in Abänderung von Beispiel 1 - nun 5 Liter pro Stunde Wasser mit niedriger Leitfähigkeit gepumpt.

Als Trägergas wird Stickstoff, der frei von CO₂ und SO₂ ist aus einem Druckbehälter benutzt, das mit 100 Liter pro Stunde in den Reaktionsbehälter und von dort in das Absorptionsgefäß geleitet wird. Das Trägergas wird nach dem Verlassen des Absorptionsgefäßes verworfen.

Die Leitfähigkeit von 0,1 µS/cm erhöht sich nach der Probenzugabe auf 2,75 µS/cm und geht anschließend wieder auf 0,1 µS/cm zurück. Das Flächenintegral unter dem Peak sowie die Peakhöhe sind ein Maß für den Sulfitgehalt der untersuchten Suspension. Die Spuren von Carbonat, die in CO₂ umgewandelt wurden, sind in diesem Fall vernachlässigbar.

Der Nachweis von Sulfit in der Suspension über die Veränderung der Leitfähigkeit des Wassers im Absorptionsgefäß wird durch Salzionen (Chlorid) in der Suspension nicht gestört.

### Beispiel 4: Kontinuierliche Bestimmung des Ammoniakgehaltes eines salzhaltigen Kondensats

Das Kondensat hat folgende Zusammensetzung:
2 mg/l Ammoniak (NH₃)
10 mg/l Natriumchlorid (NaCl)
4 mg/l Kieselsäure (SiO₂)
mehrere Salze, die mit Natronlauge (NaOH)
keine flüchtigen Verbindungen ergeben.

Die Flüssigkeit im Reaktionsbehälter wird durch kontinuierliches Eingeben von einprozentiger Natronlauge auf einem pH-Wert oberhalb 12 gehalten. Das Kondensat wird kontinuierlich mit 10 Liter pro Stunde in den Reaktionsbehälter eingegeben.

Vor Beginn der Untersuchung werden das Trägergas (Luft) mit 150 Liter pro Stunde und Wasser mit niedriger Leitfähigkeit mit 2 Liter pro Stunde - jeweils über einen NH₃-Absorber (28) beziehungsweise einen Ionenaustauscher (29) - etwa 5 Minuten lang im Kreislauf gefahren.

Wenige Sekunden nach Beginn des Eingebens des Kondensatstromes in den Reaktionsbehälter beginnt die Leitfähigkeit von 0,1 µS/cm zu steigen; nach etwa 2 Minuten hat sie sich auf einen konstanten Wert von 1,5 µS/cm eingestellt, der ein Maß für die Konzentration von Ammoniak im Kondensat ist. Während dieser Untersuchung werden das Trägergas beziehungsweise das Wasser im Absorptionsbehälter ständig mit einem konstanten Strom durch den NH₃-Absorber (28) beziehungsweise durch den Ionenaustauscher (29) gefahren.

Falls im weiteren Verlauf dieser Untersuchung die Leitfähigkeit des Wassers im Absorptionsgefäß schwankt, ist die Schwankung ein Maß für die Schwankung des NH₃-Gehalts im Kondensat.

Dieser Nachweis des Ammoniak-Gehaltes im Kondensat über die Veränderung der Leitfähigkeit des Wassers im Absorptionsgefäß wird durch Salzionen im Kondensat nicht gestört.

### Beispiel 5: Bestimmung des Carbonat-Verbrauches eines Fabrikationsabwassers (Peakauswertung)

Ein schwarzes, undurchsichtiges Fabrikationsabwasser enthält mehrere organische Säuren, Reste von Ruß und verschiedene dispergierte oder aufgeschwemmte Verunreinigungen. Ein derartiges Abwasser läßt sich nur mit aufwendiger und zeitraubender Vorbehandlung für die Untersuchung mit herkömmlichen Methoden aufbereiten. Die Messung des pH-Wertes dieser Abwasser mittels einer pH-Elektrode ist nur für kurze Zeit möglich, weil die Elektrode nach wenigen Stunden einen falschen Wert anzeigt und erst nach Reinigung eventuell weiterbenutzt werden kann.

Zur schnellen Ermittlung des Carbonatverbrauchs geht man wie folgt vor:

Eine Probe von 85 ml vorher gut gerührten Abwassers wird in den etwa 200 ml vollentsalztes Wasser enthaltenden Reaktionsbehälter gegeben. Anschließend wird eine wäßrige Lösung mit etwa 10 g/l Natriumcarbonat (Na₂CO₃) in den Reaktionsbehälter eingegeben, und zwar so lange, bis die Gasentwicklung nachweislich beendet ist, was man am Verlauf der Leitfähigkeit des Wassers im Absorptionsgefäß erkennt.

Analog zu Beispiel 1 wird das Trägergas im Kreislauf durch das Absorptionsgefäß und einen CO₂-Absorber (28) gepumpt. Das Wasser im Absorptionsgefäß wird durch einen Ionenaustauscher (29) im Kreislauf gepumpt.

Wenige Sekunden nach dem Beginn des Eingebens der Carbonat-Lösung in den Reaktionsbehälter beginnt die Leitfähigkeit des Wassers im Absorptionsgefäß zu steigen; sie erreicht nach etwa 2 Minuten ein Maximum von 9,2 µS/cm und geht nach weiteren etwa 5 Minuten auf den Grundwert von 0,1 µS/cm zurück. Die Fläche unter dem Peak ist ein Maß für den Carbonatverbrauch des Fabrikationsabwassers.

Auch diese Untersuchung kann analog zu den obengenannten Beispielen kontinuierlich durchgeführt werden. Diese Bestimmung des Carbonatverbrauchs wird weder durch Ruß noch durch Verunreinigungen gestört.

In diesem Beispiel entsteht die flüchtige Verbindung (CO₂) der eingegebenen Na₂CO₃-Lösung.

## Patentansprüche

1. Verfahren zum Nachweis mindestens einer Verbindung, die in einer mindestens einen Feststoff enthaltenden flüssigen Phase vorliegt, angewendet auf flüssige Phasen, wobei
- ein Teil der mindestens einen Feststoff enthaltenden flüssigen Phase in einen Reaktionsbehälter (1) gegeben wird,
- in den Reaktionsbehälter Chemikalien eingegeben werden,
- zumindest eine nachzuweisende Verbindung im Reaktionsbehälter in eine flüchtige Verbindung umgewandelt wird,
- mindestens eine der im Reaktionsbehälter vorhandenen flüchtigen Verbindungen mittels eines Trägergases ausgetrieben wird,
dadurch gekennzeichnet,
daß eine definierte Probenmenge der zu untersuchenden flüssigen Phase mit der im Kreis gepumpten Flüssigkeit des Reaktionsbehälters aus einer Probeentnahmevorrichtung in den Reaktionsbehälter gespült wird und daß das die flüchtigen Verbindungen enthaltende Trägergas kontinuierlich in ein Absorptionsgefäß (7) übergeleitet wird, zumindest eine der im Trägergas enthaltenen flüchtigen Verbindungen in einem Absorptionsgefäß in Wasser niedriger Leitfähigkeit absorbiert wird und die im Wasser enthaltende flüchtige Verbindung, die ein Maß für die in der mindestens einen Feststoff enthaltenden flüssigen Phase vorliegende nachzuweisende Verbindung ist, durch Messen der Leitfähigkeit oder mittels einer ionensensitiven Elektrode kontinuierlich nachgewiesen wird.

2. Verfahren nach Anspruch 1,
dadurch gekennzeichnet,
daß zum Eingeben der definierten Probemenge ein umschaltbarer, mit vier Anschlüssen ausgestatteter Durchgangshahn (33), der ein drehbares Küken mit einem bestimmten freien Volumen besitzt, das zunächst von der zu untersuchenden flüssigen Phase durchströmt wird, umgeschaltet wird und die definierte Probemenge von der im Kreis gepumpten Flüssigkeit des Reaktionsbehälters aus dem Küken in den Reaktionsbehälter gespült wird.

3. Verfahren nach den Ansprüchen 1 oder 2,
dadurch gekennzeichnet,
daß die zur Probenahme der zu untersuchenden flüssigen Phase aus einer die zu untersuchende flüssige Phase führenden Leitung abzweigende Leitung (32), welche einen Durchgangshahn (33) aufweist, zwischen zwei Probenahmen - vorzugsweise im Gegenstrom - gespült wird.

4. Verfahren nach zumindest einem der Ansprüche 1 bis 3,
dadurch gekennzeichnet,
daß die flüssige Phase im Reaktionsbehälter zur Entfernung an der Reaktion nicht teilnehmender Feststoffe über zumindest einen Filter im Kreislauf umgepumpt wird.

5. Verfahren nach zumindest einem der Ansprüche 1 bis 4, gekennzeichnet durch
- Umwandeln der nachzuweisenden Verbindung durch Eingeben von Säure, Lauge, Salz, Oxidationsmittel und/oder Reduktionsmittel,
- Austreiben von Kohlendioxid, Schwefeldioxid, Ammoniak, Stickstoffmonoxid, Stickstoffdioxid, Blausäure, Phosphorwasserstoff, Schwefelwasserstoff, Chlor, Fluor als flüchtiger Verbindung, mittels Luft, Stickstoff, Wasserstoff oder Edelgas als Trägergas.

6. Verfahren nach zumindest einem der Ansprüche 1 bis 5, gekennzeichnet durch
- Eingeben von Chemikalien in flüssiger Form in den Zulauf für das Trägergas (38) und Verteilen mittels des Trägergases in der im Reaktionsbehälter enthaltenen Flüssigkeit.

7. Verfahren nach zumindest einem der Ansprüche 1 bis 6, gekennzeichnet durch
- Einstellen der Temperatur im Reaktionsbehälter auf Werte zwischen Raumtemperatur und Siedetemperatur der Flüssigkeit im Reaktionsbehälter.

8. Verfahren nach zumindest einem der Ansprüche 1 bis 7, gekennzeichnet durch
- Eingeben mindestens einer Chemikalie in den Reaktionsbehälter, die die Umwandlung mindestens einer Verbindung in deren flüchtige Form verhindert.

9. Verfahren nach zumindest einem der Ansprüche 1 bis 8, gekennzeichnet durch
- Überleiten der Gasphase in das Absorptionsgefäß und Rückführen des Trägergases nach Verlassen des Absorptionsgefäßes in den Reaktionsbehälter, wobei gegebenenfalls Reste der flüchtigen Verbindung aus dem Trägergas abgetrennt werden.

10. Verfahren nach zumindest einem der Ansprüche 1 bis 9, gekennzeichnet durch
- Umpumpen des im Absorptionsgefäß befindlichen Wassers über einen Ionenaustauscher (29) und Rückführen des den Ionenaustauscher verlassenden Wassers in das Absorptionsgefaß,
- Einstellen der Umpumpmenge des Wassers auf eine Geschwindigkeit im Ionenaustauscher von Null Meter pro Stunde bis 100 Meter pro Stunde.

11. Verfahren nach zumindest einem der Ansprüche 1 bis 10,
dadurch gekennzeichnet,
daß das Flächenintegral der peakförmigen Leitfähigkeitserhöhung zur Auswertung des Gehalts der nachzuweisenden Verbindung in der zu untersuchenen flüssigen Phase bestimmt wird.

12. Verfahren nach zumindest einem der Ansprüche 1 bis 11,
dadurch gekennzeichnet,
daß die Peakhöhe der peakförmigen Leitfähigkeitserhöhung zur Auswertung des Gehalts der zu untersuchenden Verbindung in der zu untersuchen flüssigen Phase bestimmt wird.

13. Verfahren nach zumindest einem der Ansprüche 1 bis 12,
dadurch gekennzeichnet,
daß die zu untersuchende mindestens einen Feststoff enthaltende flüssige Phase eine Waschsuspension aus einer Rauchgasreinigunganlage ist und ein Teil dieser Waschsuspension zum Nachweis des Carbonat- oder Sulfitgehalts in den Reaktionsbehälter gegeben wird.

14. Verfahren nach zumindest einem der Ansprüche 1 bis 13 zum Nachweisen des Carbonatgehalts und/oder Sulfitgehalts einer Waschsuspension, als wäßrige Phase, aus einer Rauchgasreinigungsanlage, gekennzeichnet durch
- diskontinuierliches Eingeben einer Teilmenge der Suspension in einen Reaktionsbehälter, der mit saurem Wasser oder mit Wasser, welches Phosphorsäure enthält, gefüllt ist, das zusätzlich ein Oxidationsmittel, vorzugsweise Wasserstoffperoxid, enthält, oder
- diskontinuierliches Eingeben einer Teilmenge der Suspension in einen Reaktionsbehälter, der mit saurem Wasser oder mit Wasser, welches Phosphorsäure enthält, gefüllt ist, und
- diskontinuierliches Eingeben einer Teilmenge der Suspension durch Einspülen der Teilmenge in einen Reaktionsbehälter durch Umpumpen der Reaktionsbehälterflüssigkeit durch das Probenahmesystem,
- Automatisieren des gesamten Analysenablaufes durch die Anwendung einer freiprogrammierten Steuerung.

15. Vorrichtung zum Nachweis mindestens einer Verbindung, die in einer mindestens einen Feststoff enthaltenden flüssigen Phase vorliegt, umfassend mindestens
- einen Reaktionsbehälter (1)
- ein Absorptionsgefäß (7)
- eine Trägergasleitung (6) vom Reaktionsbehälter zum Absorptionsgefäß und
- eine dem Reaktionsbehälter (1) vorgeschaltete Probenahmevorrichtung mit einem umschaltbaren Durchlaufhahn (33), der von der im Kreis gepumpten Flüssigkeit des Reaktionsbehälters durchspült wird.

16. Vorrichtung nach Anspruch 15, umfassend weiter
- eine Analysiervorrichtung, die aus einem Absorptionsgefäß (7) besteht, das Wasser mit niedriger Leitfähigkeit enthält, in das eine Leitfähigkeitselektrode eintaucht.

17. Vorrichtung nach Anspruch 16, umfassend weiter
- einen Wasserkreislauf, der einen Ionenaustauscher (29) enthält.

18. Vorrichtung nach zumindest einem der Ansprüche 15 bis 17, umfassend weiter
- eine Trägergasleitung (23) vom Absorptionsgefäß (7) zum Reaktionsbehälter (1).

19. Vorrichtung nach Anspruch 18, umfassend weiter
- eine Vorrichtung (28) zum Abtrennen von Resten der flüchtigen Verbindung aus dem Trägergas.

## Claims

1. A process for determining at least one compound in a liquid phase containing at least one solid, applied to liquid phases, where
- some of the liquid phase containing at least one solid is introduced into a reaction vessel (1),
- chemicals are introduced into the reaction vessel,
- at least one compound to be determined is converted into a volatile compound in the reaction vessel,
- at least one of the volatile compounds present in the reaction vessel is expelled by means of a carrier gas,
characterized in that a defined amount of a sample of the liquid phase to be analyzed is rinsed out of a sampling device into the reaction vessel using the pump-circulated liquid in the reaction vessel, and in that the carrier gas containing the volatile compounds is transferred continuously into an absorption vessel (7), at least one of the volatile compounds present in the carrier gas is absorbed in low-conductivity water in an absorption vessel, and the volatile compound present in the water, which is a measure of the compound to be determined in the liquid phase containing at least one solid, is determined continuously by measurement of the conductivity or by means of an ion-sensitive electrode.

2. A process according to claim 1, characterized in that, in order to introduce a defined amount of sample, a switchable stop cock (33) fitted with four connections and having a rotatable plug with a certain free volume, through which the liquid phase to be analyzed initially flows, is switched over, and the defined amount of sample of the pump-circulated liquid in the reaction vessel is rinsed out of the plug into the reaction vessel.

3. A process according to either of claims 1 and 2, characterized in that the line (32), which, for sampling of the liquid phase to be analyzed, branches off from a line carrying the liquid phase to be analyzed and which has a stop cock (33), is rinsed between two sampling operations - preferably in countercurrent.

4. A process according to at least one of claims 1 to 3, characterized in that the liquid phase in the reaction vessel is pumped through at least one filter in order to remove solids which do not participate in the reaction.

5. A process according to at least one of claims 1 to 4, characterized by
- conversion of the compound to be determined by addition of acid, caustic lye, salt, oxidant and/or reducing agent,
- expulsion of carbon dioxide, sulphur dioxide, ammonia, nitrogen monoxide, nitrogen dioxide, prussic acid, phosphine, hydrogen sulphide, chlorine or fluorine as volatile compound by means of air, nitrogen, hydrogen or a noble gas as carrier gas.

6. A process according to at least one of claims 1 to 5, characterized by
- introduction of chemicals in liquid form into the feed for the carrier gas (38), and distribution in the liquid present in the reaction vessel by means of the carrier gas.

7. A process according to at least one of claims 1 to 6, characterized by
- adjustment of the temperature in the reaction vessel to a value between room temperature and the boiling point of the liquid in the reaction vessel.

8. A process according to at least one of claims 1 to 7, characterized by
- introduction into the reaction vessel of least one chemical which prevents the conversion of at least one compound into its volatile form.

9. A process according to at least one of claims 1 to 8, characterized by
- transfer of the gas phase into the absorption vessel and return of the carrier gas which has left the absorption vessel into the reaction vessel, any residues of the volatile compound being removed from the carrier gas.

10. A process according to at least one of claims 1 to 9, characterized by
- pumping of the water in the absorption vessel through an ion exchanger (29) and return of the water leaving the ion exchanger into the absorption vessel,
- adjustment of the pumped amount of water to a velocity in the ion exchanger of from zero metres per hour to 100 metres per hour.

11. A process according to at least one of claims 1 to 10, characterized in that the area integral of the peak-like increase in conductivity is determined in order to evaluate the content of compound to be determined in the liquid phase to be analyzed.

12. A process according to at least one of claims 1 to 11, characterized in that the peak height of the peak-like increase in conductivity is determined in order to evaluate the content of the compound to be analyzed in the liquid phase to be analyzed.

13. A process according to least one of claims 1 to 12, characterized in that the liquid phase to be analyzed containing at least one solid is a wash suspension from a flue-gas cleaning unit, and some of this wash suspension is introduced into the reaction vessel for determination of the carbonate or sulphite content.

14. A process according to at least one of claims 1 to 13 for determining the carbonate content and/or sulphite content of a wash suspension, as aqueous phase, from a flue-gas cleaning unit, characterized by
- discontinuous introduction of some of the suspension into a reaction vessel which has been filled with acidic water or with water containing phosphoric acid which additionally contains an oxidant, preferably hydrogen peroxide, or
- discontinuous introduction of some of the suspension into a reaction vessel which has been filled with acidic water or with water containing phosphoric acid, and
- discontinuous introduction of some of the suspension by rinsing this amount into a reaction vessel by pumping the liquid in the reaction vessel through the sampling system, and
- automation of the entire analytical procedure by using a RAM-programmed control system.

15. An apparatus for determining at least one compound in a liquid phase containing at least one solid, comprising at least
- a reaction vessel (1)
- a absorption vessel (7)
- a carrier-gas line (6) from the reaction vessel to the absorption vessel, and
- a sampling device upstream of the reaction vessel (1) having a switchable stop cock (33) through which the pump-circulated liquid from the reaction vessel is rinsed.

16. An apparatus according to claim 15, furthermore comprising
- an analysis apparatus comprising an absorption vessel (7) containing low-conductivity water into which a conductivity electrode dips.

17. An apparatus according to claim 16, furthermore comprising
- a water circuit containing an ion exchanger (29).

18. An apparatus according to at least one of claims 15 to 17, furthermore comprising
- a carrier-gas line (23) from the absorption vessel (7) to the reaction vessel (1).

19. An apparatus according to claim 18, furthermore comprising
- an apparatus (28) for separating residues of the volatile compound from the carrier gas.

## Revendications

1. Procédé de détection d'au moins un composé qui se présente dans une phase liquide contenant au moins une matière solide, appliqué aux phases liquides, dans lequel :
- on fournit une partie d'une phase liquide contenant au moins une matière solide, dans un récipient de réaction (1),
- on introduit dans le récipient de réaction, des produits chimiques,
- on transforme au moins un composé à détecter dans le récipient de réaction en un composé volatil,
- on chasse au moins un des composés volatils présents dans le récipient de réaction au moyen d'un gaz vecteur,
caractérisé en ce que
- une quantité définie d'échantillon de la phase liquide à étudier est rincée avec le liquide du récipient de réaction pompé à partir d'un dispositif de prélèvement d'échantillon dans le récipient de réaction et recyclé,
- le gaz vecteur qui contient les composés volatils est transfusé en continu dans un vase d'absorption (7),
- on absorbe au moins un des composés volatils contenus dans le gaz vecteur dans un vase d'absorption dans de l'eau de faible conductivité, et
- on détecte le composé volatil contenu dans l'eau d'une manière continue par mesure de la conductivité ou à l'aide d'une électrode sensible aux ions, pour mesurer le composé à détecter présent dans la phase liquide contenant au moins un composé solide.

2. Procédé selon la revendication 1,
caractérisé en ce qu'
- en vue de l'entrée de la quantité définie d'échantillon, on commute un robinet à deux voies équipé de quatre raccords (33), commutable par un boisseau orientable avec un volume libre déterminé traversé en premier lieu par la phase liquide à examiner, et
- on rince la quantité définie d'échantillon par le liquide du récipient de réaction, pompé et recyclé à partir du boisseau, dans le récipient de réaction.

3. Procédé selon la revendication 1 ou la revendication 2,
caractérisé en ce qu'
on rince, entre deux prises d'échantillon, de préférence à contre-courant, la conduite (32), qui possède un robinet à deux voies (33) et qui bifurque à partir d'une conduite de la phase liquide à examiner vers la prise d'échantillon de la phase liquide à examiner.

4. Procédé selon au moins une des revendications 1 à 3,
caractérisé en ce qu'
on transfère par pompage la phase liquide dans le récipient de réaction en vue de l'élimination des matières solides qui ne participent pas à la réaction, par l'intermédiaire d'au moins un filtre en circuit.

5. Procédé selon au moins une des revendications 1 à 4,
caractérisé par :
- la transformation du composé à détecter par introduction d'acide, de lessives, de sel, d'agent oxydant et/ou d'agent réducteur,
- l'expulsion du dioxyde de carbone, du dioxyde de soufre, de l'ammoniac, du monooxyde d'azote, du dioxyde d'azote, de l'acide cyanhydrique, de la phosphine, de l'hydrogène sulfuré, du chlore, du fluor en tant que composé volatil à l'aide de l'air, de l'azote, de l'hydrogène ou d'un gaz noble en tant que gaz vecteur.

6. Procédé selon au moins une des revendications 1 à 5,
caractérisé par
- l'introduction de produits chimiques sous forme liquide dans l'amenée pour le gaz vecteur (38), et
- la répartition au moyen du gaz vecteur, dans le liquide contenu dans le récipient de réaction.

7. Procédé selon au moins une des revendications 1 à 6,
caractérisé par
l'ajustement de la température dans le récipient de réaction, à des valeurs comprises entre la température ambiante et la température d'ébullition du liquide dans le récipient de réaction.

8. Procédé selon au moins une des revendications 1 à 7,
caractérisé par
l'introduction dans le récipient de réaction d'au moins un produit chimique qui empêche la transformation d'au moins un composé sous forme volatile.

9. Procédé selon au moins une des revendications 1 à 8,
caractérisé par
le passage de la phase gazeuse dans le vase d'absorption et le recyclage du gaz vecteur après départ du vase d'absorption dans le récipient de réaction, dans lequel les résidus éventuellement présents du composé volatil, sont séparés du gaz volatil.

10. Procédé selon au moins une des revendications 1 à 9,
caractérisé par
- le transvasement par pompage de l'eau qui se trouve dans le récipient d'absorption sur un échangeur d'ions (29) et le recyclage de l'eau qui quitte l'échangeur d'ions dans le vase d'absorption,
- l'ajustement de la quantité d'eau, transvasée par pompage, à une vitesse dans l'échangeur d'ions allant de zéro mètre par heure à 100 mètres par heure.

11. Procédé selon au moins une des revendications 1 à 10,
caractérisé en ce qu'
on détermine l'intégrale de la surface de l'augmentation de conductivité formant un pic, en vue de l'évaluation de la teneur du composé à détecter dans la phase liquide à examiner.

12. Procédé selon au moins une des revendications 1 à 11,
caractérisé en ce qu'
on détermine la hauteur du pic de l'augmentation de conductivité formant un pic, en vue de l'évaluation de la teneur du composé à examiner dans la phase liquide à examiner.

13. Procédé selon au moins une des revendications 1 à 12,
caractérisé en ce que
la phase liquide qui contient au moins un corps solide à examiner, est une suspension de lavage provenant d'une installation de gaz de fumée et en ce qu'une partie de cette suspension de lavage est ajoutée dans le récipient de réaction pour la détection de la teneur en carbonates ou en sulfites.

14. Procédé selon au moins une des revendications 1 à 13 de détection de la teneur en carbonates et/ou de la teneur en sulfites d'une suspension de lavage comme phase aqueuse provenant d'une installation d'épuration de gaz de fumée,
caractérisé par
- l'introduction discontinue d'une quantité partielle de la suspension dans un récipient de réaction rempli d'eau acide ou d'eau qui renferme de l'acide phosphorique, récipient qui contient en supplément un agent oxydant, de préférence du peroxyde d'hydrogène, ou
- l'introduction discontinue d'une quantité partielle de la suspension dans un récipient de réaction rempli d'eau acide ou d'eau qui renferme de l'acide phosphorique, et,
- l'introduction discontinue par rinçage d'une quantité partielle de la suspension dans un récipient de réaction, par pompage de recyclage du liquide du récipient de réaction à travers le système de prise d'échantillon,
- l'automatisation de la totalité du déroulement de l'analyse par utilisation d'une commande librement programmée.

15. Dispositif de détection d'au moins un composé qui se présente dans une phase liquide contenant au moins un corps solide, comprenant au moins :
- un récipient de réaction (1),
- un vase d'absorption (7)
- une conduite de gaz vecteur (6) allant du récipient de réaction au vase d'absorption et,
- un dispositif de prise d'échantillon préconnecté au récipient de réaction (1) avec un robinet à deux voies (33) commutable qui est lavé complètement par le liquide du récipient de réaction pompé en recyclage.

16. Dispositif selon la revendication 15,
comprenant en outre
un dispositif d'analyse consistant en un vase d'absorption (7) qui renferme de l'eau ayant une faible conductivité dans laquelle plonge une électrode de conductivité.

17. Dispositif selon la revendication 16,
comprenant en outre :
un circuit d'eau qui contient un échangeur d'ions (29).

18. Dispositif selon au moins une des revendications 15 à 17,
comprenant en outre
une conduite de gaz vecteur (23) allant du vase d'absorption (7) au récipient de réaction (1).

19. Dispositif selon la revendication 18,
comprenant en outre
un dispositif (28) pour séparer les résidus de composé volatil, du gaz vecteur.
